# EUROPEAN PATENT APPLICATION

(11) **EP 3 811 886 A1**
(43) Date of publication of application: **28.04.2021**
(21) Application number: 18923103.8
(22) Date of filing: 21.06.2018
(51) Int. Cl.: A61B 18/20, A61N 5/067, A61B 17/42

(54) **INTRAVAGINAL PART FOR COUPLING TO LASER GENERATOR**

(71) Applicant: INTERMEDIC ARFRAN, S.A., 28047 Madrid (ES)
(72) Inventor: SERRA LLUSA, Ricard, 08401 Granollers (ES)
(74) Representative: Ponti & Partners, S.L.P
(86) International application number: PCT/ES2018/070442
(87) International publication number: WO 2019/243641

(57) **Abstract**

The invention relates to an electromechanical intravaginal device specifically designed for a doctor to insert it inside the vagina in order to treat a specific area of the vaginal canal, achieving the stimulation and regeneration of vaginal tissue. The intravaginal device is coupled to a laser generator that produces a laser beam which crosses internally the intravaginal device and is reflected by a mirror towards the area to be treated in the vaginal canal.

It is characterised in that it allows the laser generator to fire and then the intravaginal device to rotate automatically to the following angular position successively, until reaching the predefined angular path, while at the same time advancing axially (forward or backward) within the vaginal canal in a fully automated and programmable manner, which allows fractioning the laser beam. This allows performing a great variety of treatments of the vaginal canal depending on the needs of each patient (specific areas and/or specific intensities for each area). It is also highly ergonomic for the doctor, provides a high safety for the patient (since all points and areas to treat will be treated only once), and high efficiency of treatment (since human errors are eliminated and a fully uniform and orderly delivery of the laser energy is ensured, preventing superposition of impacts and heat accumulation in a given area, as well as preventing blank spaces in the vaginal tissue).

## Description

The invention relates to an electromechanical intravaginal device specifically designed for a doctor to insert it in the vagina to treat vaginal tissue selectively in a specific area, or as a whole in the entire vaginal conduct.

The intravaginal device for treating the internal tissue of the vagina is couplable to a laser apparatus, and more specifically to a laser generator, such as a CO₂ laser generator (that is, of the type that use a CO₂ laser). Said laser beam allows treating the internal tissue of the vagina, either selectively or in its entire extension, and as a result of said treatment, for example, retracting the pelvic floor in order to raise the bladder with urine so that the change in angle thereof solves or alleviates minor or moderate urine leakage, or to make microburns in the mucous tissue so that, upon reepithelialisation, a more hydrated and terse mucous tissue is obtained.

### Background of the invention

Treatment of urine leakage, also referred to as urinary incontinence, is a problem suffered by people, and more specifically by women. With a correct diagnosis it can be remedied by various types of treatment, some of which are surgical in nature (inserting a mesh) while others are non-invasive (rehabilitation).

Urinary incontinence is a popular and generic term that encompasses several types and degrees of urine loss. Some specialists have classified it into stress incontinence, experimented when coughing, sneezing, or whenever performing a physical effort; urge incontinence, when a woman feels a sudden and intense need to urinate, and the urine is released even before reaching a toilet; mixed incontinence, which is a combination of the former two types; and finally overflow incontinence, which is constant accidental leakage of small amounts of urine.

There are currently various methods of treatment for urinary incontinence, ranging from lifestyle changes such as treatment by conditioning the vesical sphincter, physiotherapy, application of devices inserted in the bladder, medication, injection of bulking agents, and finally surgery.

There are also hand-held devices that are inserted in the vagina coupled to a laser generator apparatus meant to stimulate the regeneration of vaginal tissue with the help of laser beams generated in the corresponding apparatus. This type of hand-held devices generally consist in a hollow cylindrical element -known in the trade as a speculum- that is inserted in the vagina and moved to act on different segments, reaching the entire length of the vagina. In addition to each length of segment, the cylindrical element must be rotated manually to stimulate the vagina around 360° in the various segments, performing a correct sweep. For example, the cylindrical element can be turned 30° each time a beam is triggered, so that 12 turns are needed to cover the 360° of a circumferential area of the vagina. The typical length of a vagina is from 70 to 80 mm. A mirror is fitted at the distal end of the intravaginal device to reflect the laser beam and focus it on the specific area of the vagina to be treated. However, these hand-held devices do not work efficiently, as they are operated manually by a doctor in order to reach the entire length of the vagina, and at each distance of the length cover the 360°, such that this operation is slow and inevitable entails human error (such as mistakenly failing to stimulate one area of the vagina, or overtreating other areas with the risk of burns). Moreover, some of the known devices cannot perform a uniform sweep of burned points in the whole interior of the patient's vagina. In addition, some of the known devices cannot selectively treat one of the faces of the vagina such as, for example, only the anterior face thereof (e.g. the treatment of urinary incontinence), or only the posterior face of the vagina (e.g. for treatment to improve rectocele), or instead both the anterior and posterior faces to treat vaginal relaxation syndromes, or treat different areas of the vagina with different parameters.

It is therefore necessary to provide an alternative to the prior art that resolves the shortcomings thereof, by providing an intravaginal device couplable to a laser generator presenting an ideal efficiency with regard to the assurance of treating always the entire desired area of the vagina (avoiding errors of not treating certain areas, or errors of treating the same area twice), that can also selectively treat a single area of the vagina (e.g. Only the anterior or only the posterior face of the vagina), or treating different areas with different parameters, that is ergonomic for the doctor.

### Description of the Invention

The aim of the present invention is to provide an intravaginal device suitable for use coupled to a laser generator apparatus to stimulate regeneration of vaginal tissue (e.g. the pelvic floor area) with the help of a laser beam generated in the corresponding laser generator and aimed, focussed by said intravaginal device, at a specific area of treatment in the interior of the vagina, which overcomes the aforementioned drawbacks and presents the advantages described below.

As indicated above, the intravaginal device proposed by this invention is designed for use coupled to a laser apparatus that supplies a laser beam. The intravaginal device reflects the laser beam arriving from the laser apparatus to a specific point of a specific area of treatment in the interior of the vagina, each triggering of the laser beam producing a different burn point in the vaginal tissue. When the intravaginal part is rotated and the laser is triggered again, a series of microburn points are generated separated from each other following a specific predetermined pattern of points (which can be aligned in rows, columns, or diagonally). In this way the laser beam applied in the vagina is broken up, leaving an unburned area between two adjacent burn points following a predetermined burn order, where each burn has a predefined burn time and intensity according to the specific needs of each case. Each firing of the laser beam produces a microburn point in the vaginal tissue, which stimulates the tissue adjacent to said microburn point and causes its vaporisation, retraction, and/or creation of neocollagen, and consequently the stretching and regeneration of the tissue, re-establishing the pelvic floor. Depending on the size of the area of the vagina to be treated, the treatment is performed by a gradual treatment of circumferential areas around said treatment area.

To this end, the intravaginal device, in particular couplable to a laser beam generator apparatus, comprises at least the following elements:
- automatic means for rotating the speculum, which comprise:
   - a motor that converts a series of electrical pulses into discrete angular displacements of the output shaft thereof, thereby turning it a number of degrees depending on control inputs,
   - means for transmitting the rotation of the output shaft of the motor to a central shaft,
   - where the central shaft is aligned with the longitudinal axis of the device, the central shaft comprising:
      - a first internal shaft provided with longitudinal ribs on its outer surface, arranged such that it turns with the output shaft of the motor, and provided with a proximal end that is designed to be couplable to a laser generator,
      - a second shaft, threaded externally, that is arranged externally to the internal shaft, disposed such that it turns together with the latter and incorporating on its distal end retaining means onto the proximal end of the speculum to allow joining the speculum integrally with said distal end, such that the speculum rotates together with the rotation element; and
- an external casing that covers all or part of the automatic rotation means; and
- the speculum, which has an elongate configuration, is hollow on the inside, is closed on its distal end, and comprises an opening in a portion at its cylindrical surface near said distal end so that the laser beam can exit, and comprises inside a reflector mirror at an inclination of about 45° with respect to a longitudinal shaft of the speculum, said reflector mirror being located at an inner portion coincident or near the opening, and presenting at its proximal inlet means for attachment to the distal part of the central shaft.

The laser apparatus supplies a laser beam that passes through the intravaginal device of the invention and is reflected by the mirror placed at the distal end of the intravaginal device, where said mirror is meant to reflect the laser beam towards a specific area of the vagina predetermined by the device.

Preferably, the intravaginal part proposed by the invention is designed for use coupled to a CO₂ laser apparatus, although it can also be satisfactorily coupled to apparatuses that generate different types of lasers, such as fibre lasers, diode lasers or the like. Specifically, CO₂ lasers are gas lasers that use a gaseous mixture of carbon dioxide that is stimulated electrically. They generate infra-red (non-visible) light with a wavelength of 10.6 micrometres. CO₂ lasers have a relatively high efficiency and good beam quality, which makes them widely used for tissue treatment applications.

In addition, the intravaginal device of the invention incorporates a focussing lens arranged at a position perpendicular to the longitudinal axis of the intravaginal device and attached to the distal end of the second external shaft at a specific focal length.

According to one possible embodiment, the speculum is configured so that it is couplable, preferably removably, to the distal end of the rotation element. It preferably has a hollow cylindrical configuration. It can optionally have markings in the form of a ruler on its outer surface that extend along part or all of its length, so that the doctor can know the depth of the speculum inside the vaginal canal. Also preferably, the speculum has a rounded distal end (e.g. hemispherical) to prevent harming the patient in any way. This rounded distal end, according to one possible embodiment, can be unscrewed manually and presents on said distal end means for attaching the mirror, such as a screw. The screw is preferably flat and has a single face for reflecting the laser beam. It is preferably manufactured with a gold coat, as this is an ideal material for this purpose since it has a high reflectance in the specific working wavelengths of the laser spectrum (that is, the ray reflected in the mirror is transmitted almost entirely in a single angle, without absorption losses of the ray inside the material) and also withstands autoclaving, liquids and other sterilisation systems. However, materials other than gold can also be used, such as other metals, oxides or conductors in general.

In the proximal end the speculum presents attachment means to the distal part of the rotation element, which are preferably removable. For example, the removable attachment means can consist of a ball with a spring provided on the part of the external shaft that enters a small orifice made in the proximal end of the speculum. However, alternatively other technically equivalent attachment means can also be used.

The advantageous configuration of the speculum, as well as of the reflector mirror inclined at an angle of about 45° with respect to the longitudinal axis of the speculum, and of the opening, allows the laser beam pulses that exit the generator in a longitudinal direction to be deviated/reflected by about 90° with respect to the original direction of the beam, thereby falling on the surface of the vaginal canal tissue.

With regard to the motor, it is preferably an electromechanical stepper motor (also known as a step motor). This is an electromechanical device that converts a series of electrical pulses into discrete angular displacements, such that it can turn a number of degrees (step or half step) depending on the control inputs. This type of motor has the advantages of precision and repeatability with regard to positioning.

Preferably, the means for transmitting the rotation of the motor to the central shaft consist in a first gear with a diameter D1 connected to the output shaft of the motor, and a second gear with a diameter D2 greater than D1, where the second gear and the first gear are connected by a drive belt. Preferably, the first and second gears are grooved pulleys. However, other means for transmitting the rotation that are technically equivalent can be used without affecting the essence of the invention.

Optionally, incorporated on the ribbed internal rotating shaft is a safety optical disc, joined externally and integrally to said ribbed shaft, meant to reveal when the motor stops working erroneously; such that when the central shaft stops rotating the optical disc can detect the absence of motion and issue a command to stop the emission of the laser beam towards the interior of the intravaginal device.

Preferably, in addition to the proximal end of the central shaft that is configured to be couplable to the barrel of a laser beam generator, there is an air inlet incorporated in the rear part of the intravaginal device to supply air into the intravaginal device and ventilate same, as well as a power supply inlet. These inlets can be attached to a plate that is in turn attached to the casing.

Optionally an external covering element is incorporated, joined directly or indirectly to the casing, which does not turn with the central shaft but instead is fixed and provides protection to prevent the entire speculum from entering the vaginal cavity. Said external covering element presents a distal tip that is removable, for example, by a threaded union between said tip and the distal end. According to one example of embodiment, the external covering element is firmly attached to the casing by a threaded union through an additional part that also has a corresponding threaded union, the additional part being attached to the casing, for example, by tabs that are inserted in housings provided in the inner surface of the casing.

Optionally, an additional annular safety part is incorporated preferably having a square external transverse section and an inner orifice through which the second threaded shaft can pass with some clearance (so that it can turn freely), said annular safety part being fixedly attached to the inside of the casing, where the function of said annular safety part is to provide a channel for the central shaft such that it can turn but not move in a longitudinal direction.

Preferably, the casing consists of two parts connected to each other, for example, by screws or similar elements. According to one example of embodiment the casing is hollow and has the corresponding housings for the motor and for seating the central shaft.

Preferably, the casing is provided on the bottom with a handle for use by the doctor.

Preferably, the intravaginal device comprises motor control means connected to the motor, adapted to transmit to the motor one or more of the following electrical signals depending on the type of treatment selected by the doctor:
- Angular travel to perform in each area for each circumferential segment of the vaginal canal (that is, an angular rotation performed by the central shaft) according to the type of treatment selected by the doctor (for example, treating only half the area of a circumferential segment of the vaginal canal, so that the angular path must be only 180°, or for example treating the entire area of a circumferential segment, so that the angular path must be 360°); and/or
- Rotation angle of the motor shaft to move to the next angular firing position in the total angular path. This programmable rotation angle of the motor can be 30° per turn, defining 12 different angular firing positions in each 360° rotation, or 6 turns of 30° to cover only one of the two areas (anterior or posterior) of the vagina. Other rotation angles are possible, up to as many as 400 angular positions for each 360° turn; and/or
- Rotation rate of the motor shaft; and/or
- Stopping time between two consecutive turns for each firing.

During the rotation of the motor, with the thread pitch of the threaded external shaft, the central shaft moves in an axial direction at a rate of, for example, 1 mm per turn (forward or backward, depending on whether the treatment is performed from inside to outside of the vaginal canal or vice versa). Thus, in the treatment of a vaginal canal with a length of 70 mm, the central shaft will turn 70 times performing up to 400 firings in each turn. In addition to the rotation angle for each consecutive firing position, the total angular path to travel in each segment of the vagina can be programmed, where each angular path defines an area to be treated, and each area can be treated with different parameters (e.g. with a different laser beam energy of different density of points).

Moreover, the intravaginal device can incorporate control means for the laser beam that allow the doctor to define (i.e. configure) at least one of the following parameters according to the specific treatment time:
- The energy to be applied in the treated area; and/or
- The separation between the burn points (that is, the point density), which determines the number of firings per turn, firing once in one point, then again in another point, and so on until completing the entire matrix of points; and/or
- The order of each individual impact at each burn point of the pattern of points for each area to be treated.

Depending on the separation between the burn points configured by the doctor, depending on the type of treatment to be made in each area, it may be that if a configuration of burn points that are very separated is chosen there is no energy emission in every turn of the motor, but instead only in every other turn, for example.

Said laser beam control means and the motor control means are electronic means that can be parametrised by the doctor, preferably in a treatment display.

Optionally, end of stroke detectors and central shaft position detectors are incorporated. These are preferably formed by:
- Two identical electronic boards placed at the start and end of the path of the internal shaft, and
- A disc with a tab disposed at a fixed position on the proximal end of the threaded external shaft, such that the disc is detected by the electronic boards to stop/position the speculum when the tab is detected by the electronic boards.

According to one possible embodiment, the intravaginal device operates as follows to perform a treatment:
a) The doctor defines previously the parameters for the anterior face and the posterior face of the vagina depending on the type of treatment to be performed on each face (whether for vaginal relaxation syndrome, urinary incontinence of vaginal atrophy), as the intravaginal device can discriminate both faces.
b) The intravaginal device is positioned inside the vaginal canal, more specifically introducing the speculum in said canal. It is possible to start at the end of the canal (deepest area of the vagina) and move the speculum outward, or instead start at the beginning of the vagina and gradually introduce the speculum until covering the length to be treated.
c) The doctor activates the laser (placing it in "Ready" mode) and presses the button on the handle of the intravaginal device.
d) Then the controlled laser pulses are emitted, specifically designed for each treatment or indication, producing laser energy impacts on the vaginal mucosa and tissue. The energy impacts are performed according to predefined patterns defining an area of impact points perfectly arranged into rows, columns, and diagonals, with specific lengths.
e) The intravaginal device fires once in the first longitudinal segment of the vagina, and then moves one angular position (that is, turns an angle to the next position) to complete the angular firing path in a predetermined angular area of the first longitudinal segment. There can be up to 400 positions for each 360° turn.
f) At the same time as it turns, the intravaginal device moves in an axial direction by about 1 mm per turn. Thus, in a treatment of length 70 mm it will make 70 full turns, with up to 400 firings in each turn. The density of points is also configurable and defines the number of firings per turn.
g) If the doctor wants to pause the treatment when operating in fully automatic mode, the button can be released to stop the intravaginal device. To resume, the button must be pressed again. To stop the treatment after releasing the button, the unit must be placed in "Standby" (exiting the "Ready" mode).

Advantageously, this intravaginal device allows a great variety of treatments (of specific areas of the vaginal canal, with specific intensities depending on the area and/or type of patient) of the vaginal canal depending on the specific needs of the patient. It is also highly ergonomic for the doctor as it is convenient to handle and operate, and provides great safety for the patient (as there is a 100 % guarantee that all points and areas to be treated will be treated only once, not more), as well as a great efficiency of the treatment (minimising human errors and delivering the laser energy fractionally in a uniform and orderly manner, preventing superposition of the impacts and accumulation of heat in a given area, while also preventing untreated spaces in the vaginal tissue between impacts). For example, selective treatment of a single face of the vagina is possible, for example, treating only the anterior face/surface of the vagina to reduce potential laxness caused by childbirth and age (leaving the posterior face of the vagina untreated), or treating both faces with different intensities/energies for each face.

Further details and features will be made manifest throughout the description given below made with reference to the drawings accompanying the present specification, which show for purposes of illustration only and in a non-limiting sense a graphical depiction of the invention.

### Brief description of the figures

In order to better understand the description made, a set of drawings has been provided which, schematically and solely by way of non-limiting example, represents practical cases of various embodiments.
Figure 1 is a front perspective view of the intravaginal device (20) object of the invention, which shows the various comprising parts such as the speculum (2), the protective element (3), and the casing (1) with the handle (4).
Figure 2 is also a perspective view, in this case a rear view, of the intravaginal device (20) object of the invention, showing the various comprising parts thereof such as the speculum (2), the protective element (3), and the casing (1) with the handle (4).
Figure 3 is a front elevation view of the intravaginal device (20) object of the invention.
Figure 4 is an upper plan view of the intravaginal device (20) object of the invention.
Figure 5 is a lower plan view of the intravaginal device (20) object of the invention.
Figure 6 is a front perspective view of the intravaginal device (20) object of the invention without one of the halves of the protective casing, showing the various elements located inside the casing.
Figure 7 is the same arrangement as in figure 6 of the intravaginal device (20) object of the invention without one of the halves of the protective casing, in this case from a rear perspective view.
Figure 8 is the same arrangement as in figures 6 and 7 of the intravaginal device (20) object of the invention without one of the halves of the protective casing, in this case from a front elevation view.
Figure 9 is a cross section view along the line A-A of figure 4 of the intravaginal device (20) object of the invention in a specific position.
Figure 10 is an exploded view of the various components of the intravaginal device (20).
Figure 11 is a view of the intravaginal device (20) in its working position, that is, in which the speculum is inserted in the vaginal canal.

### Description of an example of embodiment

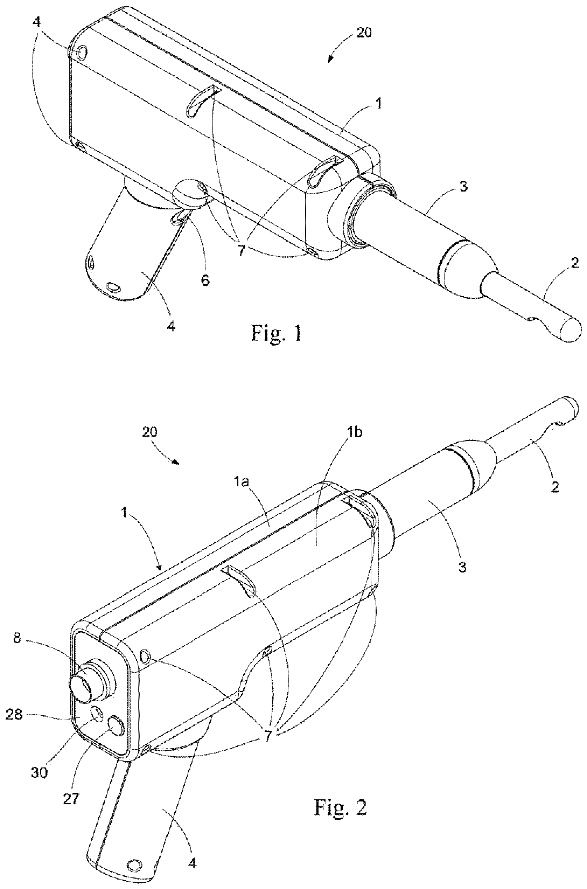

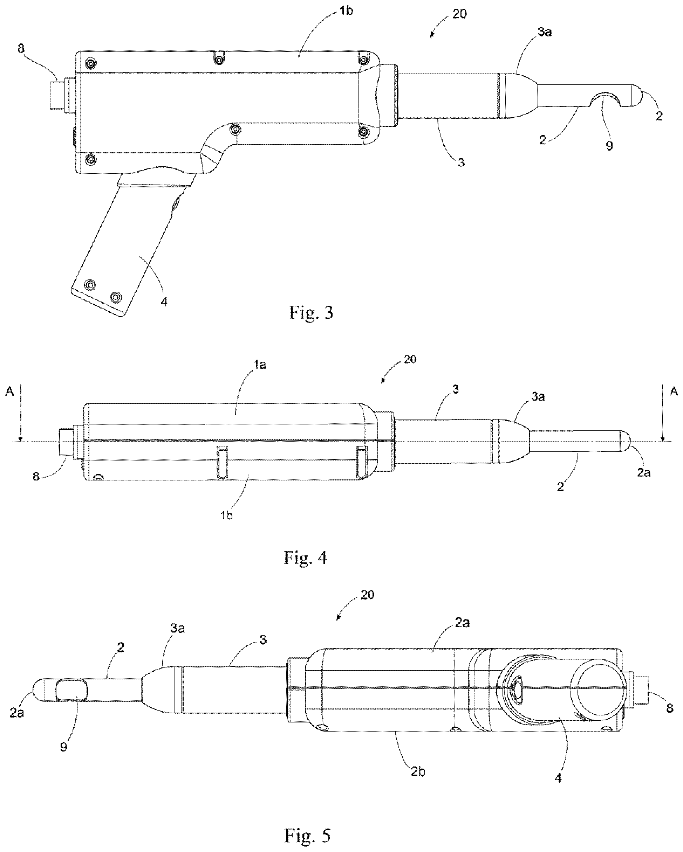

Some examples of embodiments of the intravaginal device (20) of the present invention are described below with reference to figures 1 to 11.

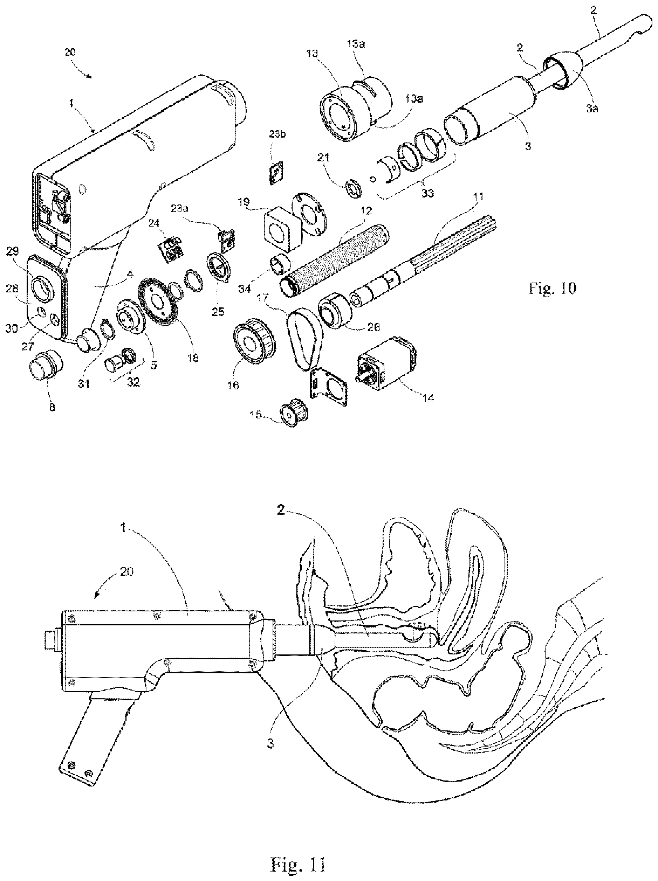

As can be seen in the accompanying figures 1 and 11, the present invention relates to an intravaginal device (20) suitable for use coupled to a laser generator equipment (not shown in the figures) to stimulate the regeneration of vaginal tissue with a laser beam in fractional mode generated in a laser generator equipment and aimed in a manner focussed by said intravaginal device (20) at a specific treatment area inside the vagina. The intravaginal device (20) of the invention comprises the following main elements:
- automatic means for rotating the speculum, which in turn comprise:
   - a motor (14) that converts a series of electrical pulses into discrete angular displacements of the output shaft thereof, such that it turns by a number of degrees that depends on control inputs,
   - means for transmission of the rotation of the output shaft of the motor (14) to a central shaft (11, 12),
   - the central shaft (11, 12) aligned with the longitudinal shaft of the device (20), said central shaft (11, 12) comprising:
      - a first internal shaft (11) having longitudinal ribs on its outer surface, arranged such that it turns with the output shaft of the motor (14), and having a proximal end (11a) that is configured to be couplable to a laser generator,
      - an externally threaded second shaft (12) arranged externally to the internal shaft (11), such that it turns together with the latter, and incorporates on its distal end (12b) means for securing it to the proximal end of the speculum (11) so that the speculum (11) can be attached to said distal end (12b), the speculum (11) thereby turning together with the rotation element (11, 12); and
- an external casing (1) that covers part or all of the automatic rotation means; and
- the speculum (2), which has an elongate configuration, is hollow on the inside, is closed on its distal end (2a), and comprises an opening (9) in a portion at its cylindrical surface near said distal end (2a) so that the laser beam can exit, and comprises inside a reflector mirror (22) at an inclination of about 45° with respect to a longitudinal shaft of the speculum (2), said reflector mirror (22) being located at an inner portion coincident or near the opening (9), and presenting at its proximal inlet means for attachment to the distal part of the central shaft (11, 12).

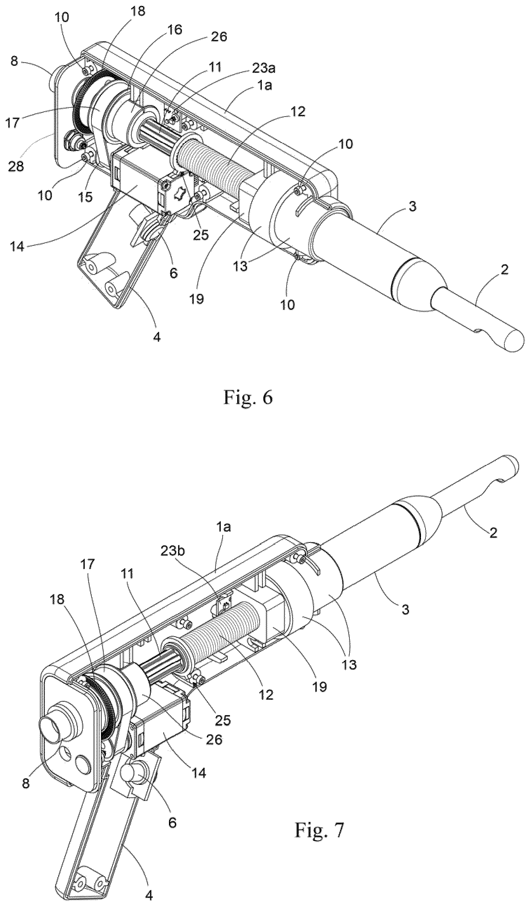

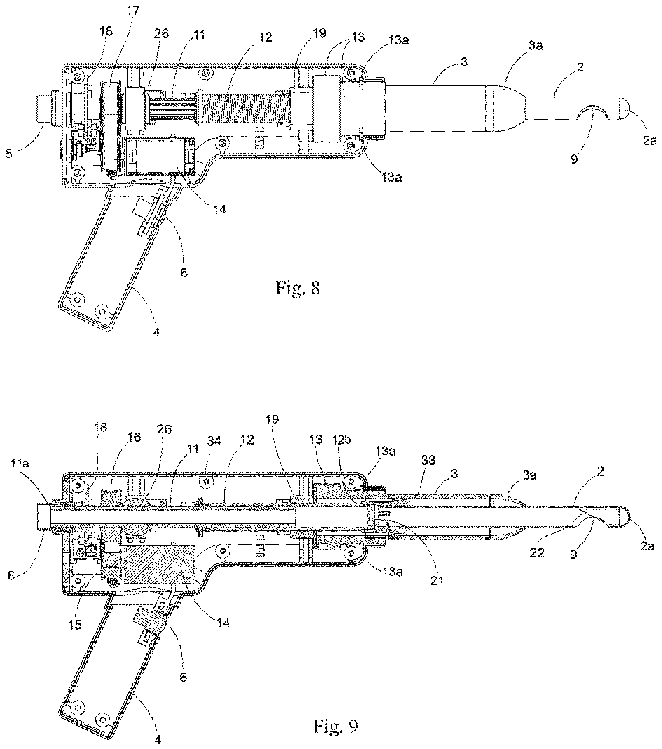

Figures 6 to 9 show all of the internal components of the intravaginal device (20). Specifically, it can be seen how the output shaft of the stepper motor (14) is connected to a first gear (15) with a diameter D1, that is connected to a second gear (16), with a diameter D2 greater than D1, by a drive belt (17). Said second gear (16) is mounted externally and joined to the first ribbed shaft (11), such that the rotation of the second gear (16) is transmitted to the first ribbed shaft (11). Mounted externally and attached to the first ribbed shaft (11) is a second externally threaded shaft (12), ending at a distal end (20) provided with a focussing lens (21). Said distal end (12) has an opening for receiving and retaining the speculum (2).
As can be seen in figure 9, this focussing lens (21) is arranged in a fixed position perpendicular to the longitudinal axis of the intravaginal device (20).

The accompanying figures show how the speculum (2) has a rounded distal end (2a) removable from the rest of the cylindrical body of the speculum (2), and also has means for attaching the mirror (22) (not shown in the figures). In the example shown, the reflector mirror (22) has a single flat face for reflecting the laser beams.

The distal end of the external shaft (12) comprises parts (33) that allow a removable attachment to the speculum (2). These consist of a protruding sphere with springs provided on the part of the external shaft (12) that is inserted in a small orifice provided in the proximal end of the speculum (2) (see figure 10).

Figure 10 shows an exploded view of all of the components present in the preferred embodiment of the intravaginal device (20). Said figure shows at the most proximal part the optical safety disc (18) mounted on the internal shaft (11) together with a sensor, where the optical safety disc (18) is externally and firmly connected to said internal shaft (11) to indicate whenever the motor (14) stops working by mistake; when the disc (18) stops rotating the sensor sends a signal to the board (24) in order to stop emitting the laser beam into the intravaginal device (20). As shown in figure 10, the disc (18) has a circumferential portion provided with homogeneous intermittent grooves in a radial direction on its entire contour, such that the sensor detects whether there is an optical interruption. In this specific embodiment the disc (18) is attached behind a support element (5).

Figures 9 and 10 show a possible configuration of the internal shaft (11). Said shaft (11) has a distal portion differentiated from the proximal portion, where the distal portion is provided with external longitudinal grooves that correspond with the internal longitudinal grooves of the intermediate part (34), where said intermediate part (34) is disposed between the internal shaft (11) and the external shaft (12).

The accompanying figures also show the external covering element (3) for the speculum (2), where said external covering element (3) is indirectly joined to the casing (1) by means of a threaded union to an additional part (13) that also has a corresponding threaded union, the additional part (13) being attached to the casing (1) by two tabs (13a) that are inserted in housings provided in the inner surface of the casing (1). Similarly to the speculum (2), the external covering element (3) has a distal tip (3a) which is removable for cleaning in each new use.

The accompanying figures also show the additional annular safety part (19) having a somewhat square-shaped external transverse section (see figure 10) and an inner orifice through which the second threaded shaft (12) can pass with some clearance, said annular safety part (19) being fixedly attached to the inside of the casing (1), where the function of said annular safety part (19) is to provide a channel for the central shaft (11, 12) such that it can turn but not move in a longitudinal direction.

The accompanying figures also show the detectors for the end of stroke and the position of the central shaft (11, 12), formed by two electronic boards (23a, 23b) placed at the start and end of the path of the central shaft (11, 12), and by a disc with a tab (25) arranged in a fixed position on the proximal end of the threaded external shaft (12), such that the disc (25) is detected by the electronic boards (23a, 23b) so as to stop/position the speculum (2) when the tab is detected by the electronic boards (23a, 23b).

The accompanying figure also show the rear connections board (28), which is attached to the most proximal part of the casing (1). This flat board presents an upper orifice (29) for coupling the laser generator and two lower orifices (30) and (27) for the passage of electrical connections and air. The connection part (8) is mounted on said upper orifice (29), externally to the rear board (18), with the help of the supporting part (31) provided internally (see figure 10), so that the laser generator (not shown) can be coupled in the inlet opening of the connection part (8). The connection parts (32) are mounted inside and outside the rear board (18) in said air orifice (27).

With regard to the casing (1), the accompanying figures show a possible configuration formed by a body that is hollow on the inside and comprising two different parts (1a, 1b) joined to each other by screws, provided with the corresponding housings for the motor and for seating the central shaft, as well as tabs for coupling the rear board (28). In order to assembly said parts (1a, 1b) together, one of the parts has housings (7) for the passage of the corresponding screws (10) and the attachment thereof in the seatings provided in the other part. The presence of a handle (4) in the lower part can also be seen on the bottom part to improve the ergonomics for the doctor when operating with this intravaginal device (20). Said handle (4) has a button (6) that the doctor must keep pressed to start firing the laser beam, stopping the firing of the laser beam when the button is released.

The elements shown in figure 10 that are not shown are auxiliary parts such as joints.

The accompanying figures only show the intravaginal device (20) in a specific position. However, the programmable and automated rotation of the motor (14) (which can have up to 400 different angular positions programmable in each 360° turn) and the thread pitch of the threaded external shaft (12) allows said central shaft (11, 12) to move in an axial/longitudinal direction (forward or backward) at a rate of, for example, 1 mm per turn.

The control means for the laser beam of the intravaginal device (20), which allow the doctor, in view of the specific treatment, to define one or more of the following parameters: energy to apply in the treated area; and/or separation between the burn points; and/or order of impact at each burn point of each pattern of points in each area to be treated, and not shown in the accompanying figures.

Figure 11 shows a possible embodiment of the intravaginal device (20) in its working position, that is, in which the speculum (2) is inserted in the vaginal canal for starting the treatment. In addition, in this first segment of the treatment the laser beam is also shown leaving the central part of the mirror (22) in a direction perpendicular to the longitudinal direction of the device.

Although reference has been made to a specific embodiment of the invention, it is clear to a person skilled in the art that the intravaginal device for coupling to a laser generator described is susceptible to numerous variations and modifications, and that all the details mentioned can be replaced by others that are technically equivalent, without departing from the scope of protection defined by the accompanying claims.

## Claims

1. An intravaginal device (20) for coupling to a laser generator, where the laser generator provides a laser beam that passes internally through the intravaginal device (20) and is reflected by at least one reflector mirror (22) placed at the most distal part of the intravaginal device (20), where said reflector mirror (22) reflects the laser beam toward a point of a specific treatment area inside the vagina, said laser beam producing a burn point in the vaginal tissue, where several microburn points separated from each other form a specific pattern of homogeneous points that can be aligned in rows, columns, or diagonally, such that said pattern of points stimulates the tissue adjacent to said microburn points causing its vaporisation, retraction, and/or creation of neocollagen, and consequently the stretching and regeneration of tissue in said treated area, **characterised in that** the intravaginal device (20) comprises:
- automatic means for rotating the speculum, which comprise:
- a motor (14) that converts a series of electrical pulses into discrete angular displacements of the output shaft thereof, such that it turns by a number of degrees that depends on control inputs,
- means for transmission of the rotation of the output shaft of the motor (14) to a central shaft (11, 12),
- the central shaft (11, 12) aligned with the longitudinal shaft of the device (20), said central shaft (11, 12) comprising:
- a first internal shaft (11) having longitudinal ribs on its outer surface, arranged such that it turns with the output shaft of the motor (14), and having a proximal end (11a) that is configured to be couplable to a laser generator,
- an externally threaded second shaft (12) arranged externally to the internal shaft (11), such that it turns together with the latter, and incorporates on its distal end (12b) means for securing it to the proximal end of the speculum (11) so that the speculum (11) can be attached to said distal end (12b), the speculum (11) thereby turning together with the rotation element (11, 12); and
- an external casing (1) that covers part or all of the automatic rotation means; and
- the speculum (2), which has an elongate configuration and is hollow on the inside, is closed on its distal end (2a), and comprises an opening (9) in a portion at its cylindrical surface near said distal end (2a) so that the laser beam can exit, and comprises inside a reflector mirror (22) at an inclination of about 45° with respect to a longitudinal shaft of the speculum (2), said reflector mirror (22) being located at an inner portion coincident or near the opening (9), and presenting at its proximal inlet means for attachment to the distal part of the central shaft (11, 12).

2. The intravaginal device (20) according to claim 1, **characterised in that** the distal end of the external shaft (12) is configured to be removably couplable to the speculum (2) by attachment means such as a ball with a spring provided on the part of the external shaft (12) that is inserted in a small orifice provided in the proximal end of the speculum (2).

3. The intravaginal device (20) according to claim 1 or 2, **characterised in that** it is configured to be used coupled to a CO₂ laser equipment.

4. The intravaginal device (20) according to any one of the preceding claims, **characterised in that** it comprises a focussing lens (21) in a position perpendicular to the longitudinal axis of the intravaginal device (20) and attached to the distal end of said second external shaft (12).

5. The intravaginal device (20) according to any one of the preceding claims, **characterised in that** the speculum (2) has a rounded and removable distal end (2a), and also has means for attaching the mirror (22).

6. The intravaginal device (20) according to any one of the preceding claims, **characterised in that** the speculum (2) has annular markings in the form of a ruler on its outer surface that extend along part or all of the length thereof.

7. The intravaginal device (20) according to any one of the preceding claims, **characterised in that** the reflector mirror (22) has a single flat face for reflecting the laser beams.

8. The intravaginal device (20) according to any one of the preceding claims, **characterised in that** the motor (14) is an electromechanical stepper motor.

9. The intravaginal device (20) according to any one of the preceding claims, **characterised in that** the means for transmitting the rotation of the motor (14) to the central shaft consist in a first gear (15) with a diameter D1 connected to the output shaft of the motor, a second gear (16) with a diameter D2 greater than D1, the second gear (16) and the first gear (15) being connected by a drive belt (17).

10. The intravaginal device (20) according to any one of the preceding claims, **characterised in that** an optical safety disc (18) can be mounted on the internal shaft (11) together with a sensor, where the optical safety disc (18) is externally and firmly connected to said internal shaft (11) to indicate whenever the motor (14) stops working by mistake, and when the disc (18) stops rotating the sensor will send a signal to stop the emission of the laser beam into the intravaginal device (20).

11. The intravaginal device (20) according to any one of the preceding claims, **characterised in that** an external covering element (3) for the speculum (2) can be incorporated, where the external covering element (3) is joined directly or indirectly to the casing (1), and where the external covering element (3) is fixed (that is, does not turn with the central shaft (11, 12)).

12. The intravaginal device (20) according to the preceding claim, **characterised in that** the external covering element (3) has a removable distal tip (3a).

13. The intravaginal device (20) according to claim 11 o 12, **characterised in that** the external covering element (3) is fixedly joined to the casing (1) by means of a threaded union to an additional part (13) that also has a corresponding threaded union, where the additional part (13) is attached to the casing (1) by, for example, tabs (13a) that are inserted in housings provided in the inner surface of the casing (1).

14. The intravaginal device (20) according to any one of the preceding claims, **characterised in that** it incorporates an additional annular safety part (19) having a somewhat square-shaped external transverse section and an inner orifice through which the second threaded shaft (12) passes with some clearance, said annular safety part (19) being fixedly attached to the inside of the casing (1), where the function of said annular safety part (19) is to provide a channel for the central shaft (11, 12) such that it can turn but not move in a longitudinal direction.

15. The intravaginal device (20) according to any one of the preceding claims, **characterised in that** the casing (1) is hollow and can be formed by two parts (1a, 1b) joined to each other, for example, by screws or other similar elements, and is provided with the corresponding housings for the motor and for seating the central shaft.

16. The intravaginal device (20) according to any one of the preceding claims, **characterised in that** the casing (1) is provided on the bottom with a handle (4).

17. The intravaginal device (20) according to any one of the preceding claims, **characterised in that** it comprises control means for the motor, connected to the motor (14), adapted so that depending on the type of treatment selected by the doctor one or more of the following electrical signals is transmitted to the motor (14):
- Angular travel to perform in each area for each circumferential segment of the vaginal canal (that is, an angular rotation performed by the central shaft) according to the type of treatment selected by the doctor (for example, treating only half the area of a circumferential segment of the vaginal canal, so that the angular path must be only 180°, or for example treating the entire area of a circumferential segment, so that the angular path must be 360°); and/or
- Rotation angle of the motor shaft to move to the next angular firing position in the total angular path. This programmable rotation angle of the motor can be 30° per turn, defining 12 different angular firing positions in each 360° rotation, or 6 turns of 30° to cover only one of the two areas (anterior or posterior) of the vagina. Other rotation angles are possible, up to as many as 400 angular positions for each 360° turn; and/or
- Rotation rate of the motor shaft; and/or
- Stopping time between two consecutive turns for each firing.

18. The intravaginal device (20) according to the preceding claim, **characterised in that** the programmable rotation angle of the motor (14) can be 30° in each rotation, thereby defining 12 angular firing positions for each 360° turn, and can have up to 400 different angular positions for each 360° turn.

19. The intravaginal device (20) according to claim 17, **characterised in that** the thread pitch of the threaded external shaft (12) allows said central shaft to move in an axial direction (forward or backward) at a rate of, for example, 1 mm per turn.

20. The intravaginal device (20) according to any one of the preceding claims, **characterised in that** it also incorporates means for controlling the laser beam which allow the doctor to define at least one or more of the following parameters depending on the specific type of treatment:
- The energy to be applied in the treated area; and/or
- The separation between the burn points (that is, the point density), which determines the number of firings per turn, firing once in each point, then again in another point, and so on until completing the entire matrix of points; and/or
- The order of impact at each burn point of each pattern of points for each area to be treated.

21. The intravaginal device (20) according to any one of the preceding claims, **characterised in that** it also incorporates detectors for the end of stroke and the position of the central shaft (11, 12), formed by:
- Two electronic boards (23a, 23b) placed at the start and end of the path of the internal shaft (11, 12); and
- A disc with a tab (25) disposed at a fixed position on the proximal end of the threaded external shaft (12), such that the disc is detected by the electronic boards (23a, 23b) to stop/position the speculum (2) when the tab is detected by the electronic boards (23a, 23b).
